# EUROPEAN PATENT APPLICATION

(11) **EP 4 261 838 A1**
(43) Date of publication of application: **18.10.2023**
(21) Application number: 22168352.7
(22) Date of filing: 14.04.2022
(51) Int. Cl.: G16H 20/60, G16H 20/70, G16H 50/20

(54) **HYDRATION FLUID PRESCRIBING SYSTEM**

(71) Applicant: Renal Care & Research, 1457 Walhain (BE)
(72) Inventor: Pozdzik, Agnieszka, 1420 Braine l'Alleud (BE)
(74) Representative: Gevers Patents

(57) **Abstract**

A system (1) suitable for remotely monitoring and maintaining improved hydration of a user, in particular a user suffering from a mineral metabolism disorder. The system comprises a server (2) operating over a data network (3), a user device (5) in communication with the server via the data network, a processing unit (6) operating on the user device (5), wherein the processing unit is configured to acquire user data (7) relating to the user's urine parameters, and a care provider device (8) in communication with the server via the data network, wherein the system further comprises a prescription unit (9) configured to generate a prescription (10) comprising a recommendation relating to hydration fluid intake by the user, and wherein the prescription is based on the user data (7) relating to the user's urine parameters.

## Description

### Field of the invention

The invention relates to the field of hydration fluid prescribing systems. The invention also relates to a method for remotely monitoring a user's hydration state.

### Description of prior art

With the increase of human health consciousness, the need of keeping the hydration state of an individual in a physiological desired hydration range has been recognized as critical. In that context, various systems and methods for human hydration monitoring have been described. Examples of such methods and systems are disclosed for example in US2020/0375533 or in US2008/0195061.

In order to ensure an autonomous usage, most of the known methods and systems for keeping a human hydrated are by design meant to be used without any intervention from a care provider. Accordingly, their medical applications, in particular in the context of a telemedicine approach, has been limited so far.

### Summary of the invention

It is an object of the invention to provide a system suitable for remotely monitoring and improving the hydration state of a user, for example of a user suffering from a mineral metabolism disorder.

The invention is defined by the independent claims. The dependent claims define advantageous embodiments.

According to the invention, there is provided a system comprising:
a) a server operating over a data network, and optionally connected to a database;
b) a user device in communication with the server via the data network;
c) a processing unit operating on the user device, wherein the processing unit is configured to acquire user data relating to the user's urine parameters and to transmit said user data to the server;
d) a care provider device in communication with the server via the data network and configured to obtain said user data from the server;
wherein the system further comprises a prescription unit configured to generate and to transmit to the user device a prescription comprising a recommendation relating to hydration fluid intake by the user, and wherein the prescription is based on the user data relating to the user's urine parameters.

In the context of the present invention the term "communication" is to be understood as "data communication" and may be a wired or a wireless data communication or a mix of both.

With such a system, a care provider (for example a nephrologist, urologist, clinician, or dietician) can remotely monitor the hydration state of a user, take appropriate steps to improve the hydration state of the user and/or monitor that a pre-established treatment plan is properly adhered to by the user. Further, the system of the present invention is particularly suitable for a telemedicine methodology, enabling specific activities such as telemonitoring, telehealth, telecare or teleconsultation.

In the context of the present invention, it was determined that a direct correlation exists between increased hydration fluid intake and reduction of a risk of a mineral metabolism disorder, such as the production of nephrolithiasis for example. Indeed, higher hydration fluid intake and better hydration lead to increased glomerular filtration rate resulting in higher production of urine and therefore reduce the propensity of the formation of crystals which could lead to the formation of kidney stones for example.

To the applicant's knowledge, the system of the prevent invention is the first system capable of remotely monitoring and improving hydration of a user based on user data relating to the user's urine parameters. Such parameters are believed to be more accurate and meaningful than other conventional biological parameters of a user, such as for example age, body weight, body mass index, height, for the specific purpose of making a recommendation relating to hydration fluid intake by the user. The urine parameters have been found to give a more accurate indication of a user's hydration state.

In an advantageous embodiment of the system according to the invention, the prescription unit operates on the care provider device. This embodiment is beneficial in many aspects as it allows continuous monitoring of the hydration state of an individual and continuous and preferably real-time communication and care delivery between the user and the (health)care provider.

In another advantageous embodiment of the system, the user's urine parameters for use herein are selected from the group consisting of urine volume, urine pH, urine density, urine colour, and any combinations thereof. These parameters have been found to constitute the most meaningful parameters for enabling remote monitoring and maintaining optimized hydration of a user, and therefore providing an improved recommendation relating to hydration fluid intake by the user.

According to another advantageous embodiment, the system as described herein further comprises an alarm unit configured to provide an alert, for example an alert to the user via the user device and/or an alert to the care provider via the care provider device, when one or more of the following user's urine parameters conditions are met:
a) the daily urine volume is lower than 3.0L / 24h, or lower than 2.8L / 24h, or lower than 2.6L / 24h, or lower than 2.5L / 24h, or lower than 2.4L / 24h, or lower than 2.2L / 24h, or lower than 2.0L / 24h;
b) the urine density is higher than 1.005, or higher than 1.010, or higher than 1.020, or higher than 1.030;
c) the urine pH is outside the range from 5.0 to 7.0, or outside the range from 5.5 to 6.5;
d) the urine colour scale is higher than 3 according to the Armstrong visual scale (which ranges from 1 to 8).

These urine parameters conditions have been found to constitute the most meaningful and critical conditions to monitor when it comes to triggering a proper recommendation relating to hydration fluid intake by the user, in particular a recommendation to increase the hydration fluid intake.

According to a particular embodiment, the alarm unit for use in the system of the present invention is advantageously selected from the group consisting of visual alarms, audible alarms, sensory alarms, and any combinations thereof. These particular forms of alarm units have been found to provide the most efficient alerts to raise the attention of the user and/or of the care provider, and thus enable a more responsive and dynamic adjustment of the prescription due to changing conditions. This in turn, enables a more efficient control of the adherence to a pre-established treatment plan or to an adjusted treatment plan.

In some embodiments of the system according to the invention, the prescription generated by the prescription unit further comprises a recommendation relating to the intake of a therapeutic substance by the user. This specific embodiment is particularly beneficial as it provides the care provider an additional tool to personalise and to more quickly adjust the overall recommendation by advantageously complementing the hydration fluid intake recommendation.

In a more advantageous embodiment, the therapeutic substance for use herein takes the form of a pill and is beneficially selected from the group of alkalinizing agents. More advantageously, the therapeutic substance is selected from the group consisting of sodium bicarbonate, potassium and/or magnesium citrate, lanthanum and/or calcium carbonate, calcium acetate, oxalate-degrading enzymes, and any mixtures thereof. These specific therapeutic substances are particularly suitable to adjust, more in particular to increase, the urine pH of the user in case the treatment plan requires so.

According to some embodiments of the system of the invention, the prescription generated by the prescription unit is communicated from the care provider device to the user device, in particular via the data network. This embodiment is particularly beneficial as it allows communication and care delivery between the user and the (health)care provider, which in turn enables a more efficient and dynamic control of the adherence to the pre-established treatment plan or to an adjusted treatment plan. Further, this particular embodiment is particularly efficient in the context of a telemedicine approach.

According to another advantageous embodiment, the system of the invention further comprises a hydration fluid delivery article in communication with the user device. This embodiment is particularly advantageous as it enables a more convenient adherence by the user to the pre-established treatment plan or to an adjusted treatment plan.

In some advantageous embodiments of the system, the processing unit for use herein is further configured to establish a hydration fluid consumption plan, wherein the hydration fluid consumption plan comprises a plurality of hydration fluid drinking events scheduled at predetermined intervals, wherein each hydration fluid drinking event is associated with a specific hydration fluid intake target, and wherein the processing unit is further configured to compare the hydration fluid intake with the specific hydration fluid intake target for each hydration fluid consumption event and to cause the prescription unit to generate a prescription comprising a recommendation for the user to consume more hydration fluid if the hydration fluid intake during the hydration fluid consumption event is smaller than the specific hydration fluid intake target associated with that hydration fluid consumption event, wherein the prescription is provided before the next hydration fluid consumption event.

This particular embodiment is particularly beneficial as it not only allows the care provider to establish a personalized hydration fluid consumption plan for the user, but it further enables both the user and the care provider to ensure that the hydration fluid consumption plan is adhered to in a continuous, dynamic, proactive and autonomous manner.

In some other embodiments of the system according to the invention, the prescription unit for use herein is further configured to generate a revised prescription depending on the adherence of the user to the hydration fluid consumption plan. This embodiment is particularly advantageous as it enables the care provider or the prescription unit itself to dynamically adjust the prescription, in particular the recommendation relating to the hydration fluid and/or the therapeutic substance intake by the user, and make sure that the overall hydration fluid consumption plan is still adhered to even in those cases where the initial recommendation was not respected by the user.

According to a particularly advantageous embodiment, the system of the invention is particularly suitable for remotely advising a user suffering from mineral metabolism disorders, in particular kidney stones (or nephrolithiasis) and chronic kidney diseases (from stage 1 to stage 5). This embodiment is particularly useful as it allows a care provider to advise and assist a user in managing and/or preventing mineral metabolism disorder conditions.

In one beneficial embodiment, the system of the invention further comprises any of:
a) a learning module which is in particular supported by deep machine learning protocols and algorithms;
b) an education module;
c) a (self) motivation module;
d) a reward module;
e) a quality-of-life assessment module; and
f) a diet recommendation module.

According to this specific execution, the system of the invention can further improve its capabilities and the quality of the prescription generated by the prescription unit. According to this particular execution still, the user is enabled to take a more proactive and rewarding part in the overall treatment plan, which in turns contribute to providing a more satisfying user experience.

It is another object of the invention to provide a method for remotely monitoring a user's hydration state, comprising the steps of:
a) providing a system as described above;
b) prompting the user to enter the data relating to the user's urine parameters on the user device;
c) operating the prescription unit to generate a prescription comprising a recommendation relating to hydration fluid intake by the user, and wherein the prescription is based on the data relating to the user's urine parameters entered by the user on the user device.

According to an advantageous embodiment, the method of the invention is particularly suitable for remotely advising a user suffering from mineral metabolism disorders, in particular kidney stones (or nephrolithiasis) and chronic kidney diseases (from stage 1 to stage 5).

It is yet another object of the invention to provide a computer program for causing a computer to execute a step of generating a prescription comprising a recommendation relating to hydration fluid intake which is suitable for a user, and wherein the prescription is based on data relating to the user's urine parameters.

In an advantageous embodiment of the program according to the invention, the prescription further comprises a recommendation relating to the intake of a therapeutic substance suitable for the user.

### Short description of the drawings

These and further aspects of the invention will be explained in greater detail by way of examples and with reference to the accompanying drawings in which:
- Fig.1: shows a diagram depicting a schematic configuration of a system according to an exemplary embodiment of the present invention;
- Fig.2: shows a diagram depicting a schematic configuration of a user device, a processing unit and data relating to the user's urine parameters for use in a system according to one exemplary embodiment of the present invention;
- Fig.3: shows a diagram depicting a schematic configuration of a user device, a processing unit and a hydration fluid delivery article for use in a system according to one exemplary embodiment of the present invention;
- Fig.4: shows a block diagram depicting a schematic configuration of a server for use in a system according to one exemplary embodiment of the present invention.

The drawings of the figures are neither drawn to scale nor proportioned. Generally, similar or identical components are denoted by the same reference numerals in the figures.

### Detailed description of embodiments of the invention

According to a first aspect of the invention, there is provided a system comprising:
a) a server operating over a data network, and optionally connected to a database;
b) a user device in communication with the server via the data network;
c) a processing unit operating on the user device, wherein the processing unit is configured to acquire user data relating to the user's urine parameters and to transmit said user data to the server;
d) a care provider device in communication with the server via the data network and configured to obtain said user data from the server;
wherein the system further comprises a prescription unit configured to generate and to transmit to the user device a prescription comprising a recommendation relating to hydration fluid intake by the user, and wherein the prescription is based on the user data relating to the user's urine parameters.

Reference will now be made particular embodiments of the present invention, examples of which are illustrated in the accompanying figures. The accompanying figures are intended to provide a better understanding of the embodiments. They show schematics of embodiments and serve in conjunction with the description to explain principles and concepts of the disclosed subject matter.

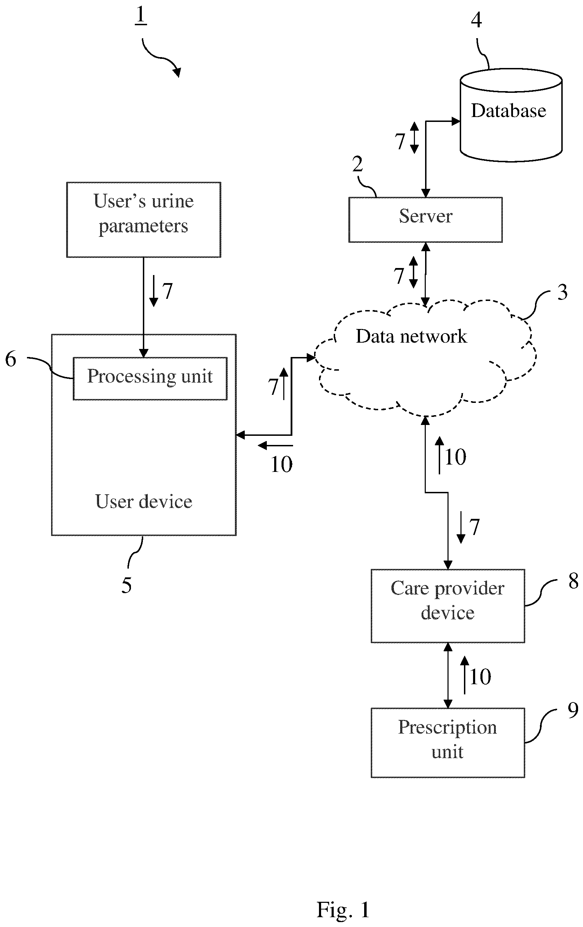

Fig.1 shows a diagram depicting a schematic configuration of a system according to an exemplary embodiment of the present invention. The system (1) comprises a server (2) operating over a data network (3), which is optionally connected to a database (4). The system (1) further comprises a user device (5) in communication with the server (2) via the data network (3), and a processing unit (6) operating on the user device (5), wherein the processing unit (6) is configured to acquire and to process user data (7) relating to the user's urine parameters and to transmit said user data (7) to the server. The system (1) further comprises a care provider device (8) in communication with the server (2) via the data network (3) to receive said user data (7) from the user, as well as a prescription unit (9) configured to generate a prescription (10) comprising a recommendation relating to hydration fluid intake by the user and wherein the prescription is based on the user data (7) relating to the user's urine parameters.

As will be apparent to those skilled in the art, a server for use herein may perform various typical operations and functions. Exemplary operations include processing and analysing various data such as user's personal data and external data, requesting input, providing instructions, sending messages and alerts. The server for use herein may be assisted with suitable computer programs, software's, algorithms, machine learning protocols and models, and may in some examples also be provided with artificial intelligence capabilities. Suitable servers for use herein are not particularly limited and will be easily identified by those skilled in the art in the light of the present disclosure.

The data networks for use herein are not particularly limited. An exemplary suitable data network is the Internet network.

Similarly, databases for use herein will be easily identified by those skilled in the art and are not particularly limited either. Databases for use herein are typically provided with suitable memory or data storage units which are typically able to store various types of data, in particular the user data (7) relating to the user's urine parameters and data relating to the prescription (10), and/or the hydration fluid intake by the user, and/or the therapeutic substance intake by the user, and/or the adherence of the user to the hydration fluid consumption plan, and/or the therapeutic substance intake plan.

The system of the invention further comprises a user device in communication with the server via the data network. A suitable user device for use herein is for example a so-called terminal device, which usually comprises appropriate data communication means to ensure proper connectivity with the data network and access to the server. In some embodiments, the user device may comprise memory or data storage units that are suitable to store various data, such as in particular user's personal data and data relating to the user's urine parameters. In some other embodiments, the user device may comprise an interactive display or interface.

Suitable user devices for use herein include, but are not limited to, smart mobile devices. Suitable smart mobile devices comprise smartphones, tablets, laptops, or any other handheld or portable connected devices. In an advantageous embodiment, the user device is a smartphone.

The system of the invention further comprises a processing unit operating on the user device, wherein the processing unit is configured to acquire and process the user data relating to the user's urine parameters. Processing units for use herein will be easily identified by those skilled in the art. A suitable processing unit is for example a microcomputer.

In an advantageous embodiment, the user device for use in the system of the invention is associated with a suitable computer program or software application. Suitable applications are for example (integrated) mobile applications or web applications running on remote or mobile web browsers.

According to the present invention, the processing unit preferably operates on the user device. However, the processing unit for use herein is not necessarily located and integrated into the user device. In some embodiments, the processing unit may be located in the server, or in any other suitable parts of the system.

In an advantageous embodiment, the user device for use herein is configured to receive, store, process and communicate user data relating to the user's urine parameters and/or to hydration fluid intake by the user.

The system of the invention further comprises a care provider device in communication with the server via the data network.

Similar to the user device as described hereinbefore, a suitable care provider device for use herein is for example a so-called terminal device, which usually comprises appropriate data communication or transmission means to ensure proper connectivity with the data network and access to the server. In some embodiments, the care provider device may comprise memory or data storage units that are suitable to receive and store various data, such as for example user's personal data and the user data (7) relating to the user's urine parameters. In some other embodiments, the care provider device may comprise an interactive display or interface.

Suitable care provider devices for use herein include, but are not limited to, personal computing devices or smart mobile devices. Suitable smart mobile devices comprise smartphones, tablet, laptops, or any other handheld or portable connected devices. In an advantageous embodiment, the user device is a personal computing device, such as a desktop or laptop computer for example.

In an advantageous embodiment, the care provider device for use in the system of the invention is associated with a suitable computer program or software application. Suitable applications are for example (integrated) desktop applications or web applications running on remote or mobile web browsers.

In a typical embodiment, a computing unit operates on the care provider device. The computing unit for use herein is not necessarily located and integrated into the care provider device. In some embodiments, the computing unit operating on the care provider device may be located in the server, or in any other suitable parts of the system.

In an advantageous embodiment, the care provider device for use herein is configured to receive, store, process and communicate user data relating to the user's urine parameters and/or to hydration fluid intake by the user.

In a particularly advantageous embodiment, the care provider is provided with a visual dashboard.

The system of the invention further comprises a prescription unit configured to generate a prescription. Prescription units for use herein will be easily identified by those skilled in the art. A suitable prescription unit is typically a microcomputer.

In a typical embodiment, the prescription unit operates on the care provider device. The prescription unit for use herein is not necessarily located and integrated into the care provider device. In some embodiments, the prescription unit operating on the care provider device may be located on the server, or in any other suitable parts of the system.

In an advantageous embodiment, the prescription unit for use herein is configured to receive, store, process and communicate user data relating to the user's urine parameters and/or to the hydration fluid intake by the user.

According to an exemplary embodiment, the prescription unit for use herein is associated or in communication with the computing unit operating on the care provider device.

In a typical embodiment, the prescription unit operates on the care provider device, and the care provider device is in communication with the server via the data network. Accordingly, the prescription (10) generated by the prescription unit is for example communicated by the care provider device to the user device, in particular via the data network. In some examples, the user's urine parameters are selected from the group consisting of urine volume, urine pH, urine density, urine colour, content of additional urine (biochemical) components, and any combinations thereof. Additional urine (biochemical) components comprise, but are not limited to, red cells, white cells, leucocytes, bacteria, urobilinogen, creatinine, urea, uric acid, sodium, chlore, potassium, calcium, oxalates, phosphate oxalate, citrates, nitrates, sulphates, glucose, proteins, and any combinations or mixtures thereof.

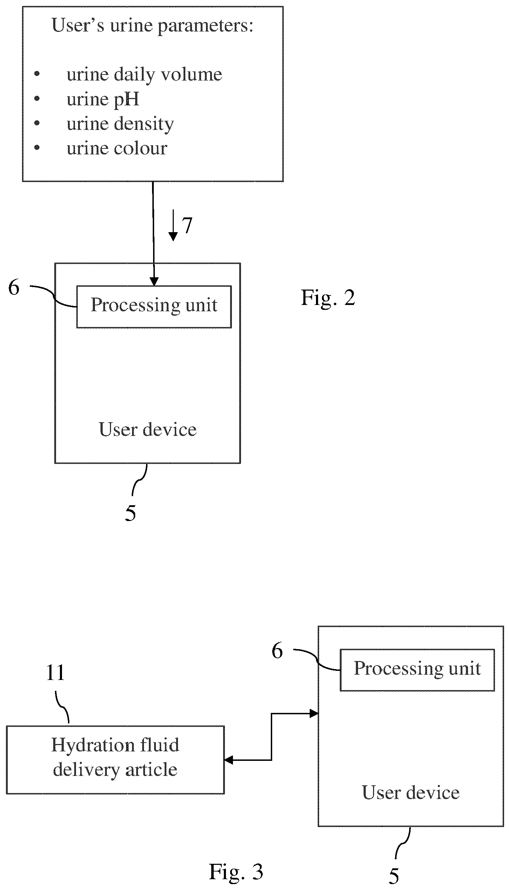

Fig.2 shows a diagram depicting a schematic configuration of a user device (5), a processing unit (6) and user data (7) relating to the user's urine parameters for use in a system (1) according to one exemplary embodiment of the present invention. In the execution shown in Fig.2, the processing unit operates on the user device (5). The user device is typically in communication with the server via the data network.

In an exemplary embodiment, the prescription unit generates a personalized prescription (10) comprising a recommendation relating to hydration fluid intake by the user to ensure an improved hydration level for the user, wherein the prescription (10) is repeatedly adapted in function of the user data relating to the user's urine parameters, and wherein those user data are received from a remote location, typically from the user device.

As such, the system of the present invention may be considered as a hydration fluid prescribing system, as the prescription comprises a recommendation relating to hydration fluid intake by the user. The disclosed system may further be referred to as a system suitable for remotely monitoring a user's hydration state and/or a user's urinary parameters. The system as described herein may further be regarded as system suitable for (re) alkalinisation of a user's urine.

According to an advantageous embodiment, the system of the present invention further comprises an alarm unit (not shown) configured to provide an alert to the user and/or to the care provider when one or more of the following user's urine parameters (unfavorable) conditions are met:
a) the daily urine volume is lower than 3.0L / 24h, or lower than 2.8L / 24h, or lower than 2.6L / 24h, or lower than 2.5L / 24h, or lower than 2.4L / 24h, or lower than 2.2L / 24h, or lower than 2.0L / 24h;
b) the urine density is higher than 1.005, or higher than 1.010, or higher than 1.020, or higher than 1.030;
c) the urine pH is outside the range from 5.0 to 7.0, or outside the range from 5.5 to 6.5;
d) the urine colour scale is higher than 3 according to the Armstrong visual scale (which ranges from 1 to 8).

Alarm units configured to provide an alert and for use in the present system are not particularly limited. Exemplary alarm units include visual alarms, audible alarms, sensory alarms, and any combinations thereof. Alarm units for use herein may be located in any suitable parts of the system.

In an advantageous embodiment, the alarm unit is located in any of the user device, the care provider device, and any combinations thereof. More advantageously, the alarm unit for use herein is located or is at least partly comprised in the user device. Typically, in those situations where the alert is provided due one or more of the above-detailed unfavourable user's urine parameters conditions being met, this would trigger a recommendation relating to hydration fluid intake, in particular a prompt to the user to increase his hydration fluid intake.

In one particularly advantageous embodiment of the system of the invention, the prescription (10) further comprises a recommendation relating to the intake of a therapeutic substance by the user. The therapeutic substance for use herein are typically meant to contribute to adjusting or modifying the user's urine parameters so that they fall within a predetermined range or to get close to a predetermined value. More particularly, the therapeutic substance is designed for none of the above-detailed user's unfavourable urine parameters conditions to be met, or none to be met anymore, after ingestion of the therapeutic substance by the user. Even more advantageously, the therapeutic substance is designed for the none of the above-detailed unfavourable user's urine parameters conditions to be met in conjunction with the hydration fluid intake.

The therapeutic substance is for example a substance which may take the form of a pill, a capsule, a tablet, a bead, drops of a liquid substance, or any combinations or mixtures thereof.

Preferably, the therapeutic substance for use herein is selected from the group consisting of alkalinizing agents, oxalate binding agents, oxalate degrading agents, phosphate binding agents, potassium binding agents, and any combinations or mixtures thereof. More preferably, the therapeutic substance is selected from the group consisting of potassium citrate, magnesium citrate, sodium bicarbonate, lanthanum carbonate, calcium carbonate, any aluminum or iron based preparation, sevelamer based preparations, sodium zirconium cyclosilicate, patiromer sorbited calcium, sucroferric oxyhydroxide, calcium acetate, reloxaliase, myo-inositol hexakisphosphate (IP-6) analogue, pH-tolerant oxalate degrading enzymes (such as e.g. Oxidien: OX-1), oxalate-degrading enzymes, rationally designed microbial consortia, genetically engineering microbial medicines, lumasiran and any combinations or mixtures thereof.

More preferably, the therapeutic substance for use herein is selected from the group of alkalinizing agents, more in particular the therapeutic substance is selected from the group consisting of sodium bicarbonate, potassium and/or magnesium citrate, lanthanum and/or calcium carbonate, calcium acetate, oxalate-degrading enzymes, and any combinations or mixtures thereof.

According to the embodiment of the system where the prescription further comprises a recommendation relating to the intake of a therapeutic substance, any of the user device, the care provider device, the prescription unit, the processing unit or the computing unit are further configured to receive, store, process and communicate data relating to the intake of the therapeutic substance.

In an advantageous embodiment of the system of the invention, the prescription unit for use herein is further configured to generate and communicate, preferably in real-time, a revised prescription based on data relating to any of the therapeutic substance intake or hydration fluid intake.

In one particular embodiment of the system, the prescription generated by the prescription unit is communicated by the care provider device to the user device, in particular via the data network.

In another beneficial embodiment, the system of the invention further comprises a hydration fluid delivery article which is in communication with the user device.

**Fig.3** shows a diagram depicting a schematic configuration of a user device (5) comprising a built-in processing unit (6) in communication with an exemplary hydration fluid delivery article (11) for use in the system (1) of the present invention.

Hydration fluid delivery articles for use herein are not particularly limited. Exemplary hydration fluid delivery articles include containers, cans, bottles, canisters or boxes.

According to another beneficial embodiment of the system, the processing unit is further configured to establish a hydration fluid consumption plan, wherein the hydration fluid consumption plan comprises a plurality of hydration fluid drinking events scheduled at predetermined intervals, wherein each hydration fluid drinking event is associated with a specific hydration fluid intake target, and wherein the processing unit is further configured to (continuously) compare the hydration fluid intake with the specific hydration fluid intake target for each hydration fluid consumption event and to cause the prescription unit to generate and communicate a prescription comprising a recommendation - for example a visual indication - for the user to consume more hydration fluid if the hydration fluid intake during the hydration fluid consumption event is smaller than the specific hydration fluid intake target associated with that hydration fluid consumption event, wherein the prescription is provided before the next hydration fluid consumption event.

Advantageously, the prescription unit for use herein is further configured to generate (and communicate), preferably in real-time, a revised prescription depending on the adherence of the user to the hydration fluid consumption plan, wherein the revised prescription is for example based on data relating to any of the therapeutic substance intake or hydration fluid intake by the user.

Advantageously still, the system of the present invention may further comprise any of:
a) a learning module which is in particular supported by artificial intelligence-based protocols and algorithms, more in particular deep machine learning;
b) an education module;
c) a (self) motivation module;
d) a reward module;
e) a quality-of-life assessment module; and
f) a diet recommendation module, in particular a recommendation module relating to low oxalate and low calcium diet.

The various additional modules for use herein may be located in any suitable parts of the system. Advantageously, the additional modules are located on the server.

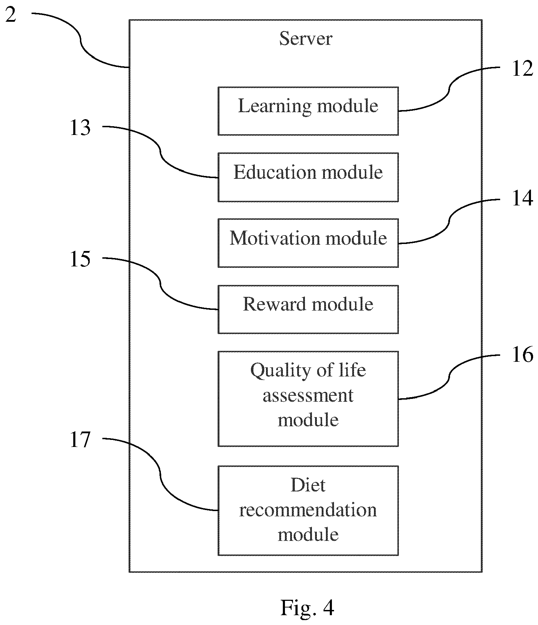

Fig.4 shows a block diagram depicting a schematic configuration of a server (2) for use in a system (1) according to the present invention, wherein the server (2) comprises a learning module (12), an education module (13), a (self) motivation module (14), a reward module (15), a quality-of-life assessment module (16), and a diet recommendation module (17).

The system of the present invention is particularly suitable for remotely monitoring and/or advising a user suffering from mineral metabolism disorders, in particular kidney stones (or nephrolithiasis) and chronic kidney diseases (from stage 1 to stage 5). The system of the present invention is also particularly suitable for remotely monitoring and/or advising a user suffering from dehydration. The system is further suitable for remotely monitoring and/or advising a user subject to dialysis and/or transplantation, and/or suffering from a heart disease.

It is another object of the invention to provide a method for remotely monitoring a user's hydration state, comprising the steps of:
a) providing a system as described above;
b) prompting the user to enter the data relating to the user's urine parameters on the user device;
c) operating the prescription unit to generate a prescription comprising a recommendation relating to hydration fluid intake, and wherein the prescription is based on the data relating to the user's urine parameters entered by the user on the user device.

In an advantageous embodiment, the method of the present invention further comprises the steps of:
d) operating the prescription unit for it to establish a personalized hydration fluid consumption plan, wherein the hydration fluid consumption plan comprises a plurality of hydration fluid consumption events scheduled at predetermined intervals, wherein each hydration fluid consumption event is associated with a specific hydration fluid intake target,
e) further operating the prescription unit to compare the hydration fluid intake with the specific hydration fluid intake targets for each hydration fluid consumption event, and
f) further operating the prescription unit to generate and communicate a prescription comprising a recommendation - in particular a visual indication - for the user to consume more hydration fluid if the hydration fluid intake during the hydration fluid consumption event is smaller than the specific hydration fluid intake target associated with that hydration fluid consumption event, wherein the prescription is provided before the next hydration fluid consumption event.

According to an advantageous embodiment, the method of the invention is particularly suitable for remotely advising a user suffering from a mineral metabolism disorder, in particular kidney stones (also called nephrolithiasis) and/or a chronic kidney disease (from stage 1 to stage 5). The method is also suitable for remotely advising a user suffering from dehydration or subject to dialysis and/or being transplanted, as well as for advising a user suffering from a heart disease.

It is yet another object of the invention to provide a computer program for causing a computer to execute a step of generating a prescription comprising a recommendation relating to a hydration fluid intake which is suitable for a user, and wherein the prescription is based on data relating to the user's urine parameters.

In an advantageous embodiment of the program according to the invention, the prescription further comprises a recommendation relating to the intake of a therapeutic substance suitable for the user.

The computer program according to the invention typically stores executable instructions, which when executed by a computer, cause the computer to execute the step of generating the prescription or the method as described above.

The present invention has been described in terms of specific embodiments, which are illustrative of the invention and not to be construed as limiting. More generally, it will be appreciated by persons skilled in the art that the present invention is not limited by what has been particularly shown and/or described hereinabove.

Reference numerals in the claims do not limit their protective scope. Use of the verbs "to comprise", "to include", "to be composed of", or any other variant, as well as their respective conjugations, does not exclude the presence of elements other than those stated. Use of the article "a", "an" or "the" preceding an element does not exclude the presence of a plurality of such elements.

The invention may also be described as follows: a system (1) suitable for remotely monitoring and maintaining improved hydration of a user, in particular a user suffering from a mineral metabolism disorder. The system comprises a server (2) operating over a data network (3), a user device (5) in communication with the server via the data network, a processing unit (6) operating on the user device (5), wherein the processing unit is configured to acquire user data (7) relating to the user's urine parameters, and a care provider device (8) in communication with the server via the data network, wherein the system further comprises a prescription unit (9) configured to generate a prescription (10) comprising a recommendation relating to hydration fluid intake by the user, and wherein the prescription is based on the user data (7) relating to the user's urine parameters.

## Claims

1. A system (1) comprising:
a) a server (2) operating over a data network (3), and optionally connected to a database (4);
b) a user device (5) in communication with the server (2) via the data network (3);
c) a processing unit (6) operating on the user device (5), wherein the processing unit (6) is configured to acquire user data (7) relating to the user's urine parameters and to transmit said user data to the server;
d) a care provider device (8) in communication with the server (2) via the data network (3) and configured to obtain said user data from the server;
**characterized in that** the system (1) further comprises a prescription unit (9) configured to generate and to transmit to the user device a prescription (10) comprising a recommendation relating to hydration fluid intake by the user, and wherein the prescription is based on the user data (7) relating to the user's urine parameters.

2. A system (1) according to claim 1, wherein the prescription unit (9) operates on the care provider device (8).

3. A system (1) according to any of claim 1 or 2, wherein the user's urine parameters are selected from the group consisting of urine volume, urine pH, urine density, urine colour, and any combinations thereof.

4. A system (1) according to claim 3, which further comprises an alarm unit configured to provide an alert when one or more of the following user's urine parameters conditions are met:
a) the daily urine volume is lower than 3.0L / 24h, or lower than 2.8L / 24h, or lower than 2.6L / 24h, or lower than 2.5L / 24h, or lower than 2.4L / 24h, or lower than 2.2L / 24h, or lower than 2.0L / 24h;
b) the urine density is higher than 1.005, or higher than 1.010, or higher than 1.020, or higher than 1.030;
c) the urine pH is outside the range from 5.0 to 7.0, or outside the range from 5.5 to 6.5;
d) the urine colour scale is higher than 3 according to the Armstrong visual scale.

5. A system (1) according to any of the preceding claims, wherein the prescription (10) generated by the prescription unit further comprises a recommendation relating to the intake of a therapeutic substance by the user.

6. A system (1) according to any of the preceding claims, wherein the prescription generated by the prescription unit is communicated by the care provider device (8) to the user device (5) via the data network (3).

7. A system (1) according to any of the preceding claims, which further comprises a hydration fluid delivery article (11) in communication with the user device (5).

8. A system (1) according to any of the preceding claims, wherein the processing unit (6) is further configured to establish a hydration fluid consumption plan, wherein the hydration fluid consumption plan comprises a plurality of hydration fluid drinking events scheduled at predetermined intervals, wherein each hydration fluid drinking event is associated with a specific hydration fluid intake target, and wherein the processing unit is further configured to compare the hydration fluid intake with the specific hydration fluid intake target for each hydration fluid consumption event and to cause the prescription unit (9) to generate a prescription (10) comprising a recommendation for the user to consume more hydration fluid if the hydration fluid intake during the hydration fluid consumption event is smaller than the specific hydration fluid intake target associated with that hydration fluid consumption event, wherein the prescription is provided before the next hydration fluid consumption event.

9. A system (1) according to claim 8, wherein the prescription unit (9) is further configured to generate a revised prescription depending on an adherence of the user to the hydration fluid consumption plan.

10. A system (1) according to any of the preceding claims, which is particularly suitable for remotely advising a user suffering from mineral metabolism disorders, in particular kidney stones and/or chronic kidney diseases.

11. A system (1) according to any of the preceding claims, which further comprises any of:
a) a learning module (12);
b) an education module (13);
c) a (self) motivation module (14);
d) a reward module (15);
e) a quality-of-life assessment module (16); and
f) a diet recommendation module.

12. A method for remotely monitoring a user's hydration state, comprising the steps of:
a) providing a system (1) according to any of the preceding claims;
b) prompting the user to enter the user data (7) relating to the user's urine parameters on the user device (5);
c) operating the prescription unit (9) to generate a prescription (10) comprising a recommendation relating to hydration fluid intake by the user, and wherein the prescription is based on the user data (7) relating to the user's urine parameters entered by the user on the user device (5).

13. A method according to claim 12, which is particularly suitable for remotely advising a user suffering from a mineral metabolism disorder, in particular kidney stones and/or a chronic kidney disease and/or a heart disease.

14. A program for causing a computer to execute a step of generating a prescription comprising a recommendation relating to a hydration fluid intake which is suitable for a user, and wherein the prescription is based on user data relating to the user's urine parameters.

15. A program according to claim 14, wherein the prescription further comprises a recommendation relating to the intake of a therapeutic substance suitable for the user.
